(19) Europäisches Patentamt
European Patent Office
Office européen des brevets

(11) **EP 3 637 068 B1**

(12) **EUROPEAN PATENT SPECIFICATION**

(45) Date of publication and mention
of the grant of the patent:
**26.04.2023 Bulletin 2023/17**

(21) Application number: **19202281.2**

(22) Date of filing: **09.10.2019**

(51) International Patent Classification (IPC):
**G01J 3/42** (2006.01)          **G01N 21/31** (2006.01)
**G01N 21/53** (2006.01)          **G01N 33/00** (2006.01)
**G01J 3/28** (2006.01)          G01N 21/3504 (2014.01)
**G01J 3/12** (2006.01)

(52) Cooperative Patent Classification (CPC):
**G01J 3/42; G01J 3/2803; G01N 21/53;**
**G01N 21/538; G01N 33/0075;** G01J 2003/1217;
G01J 2003/2806; G01N 2021/3531

(54) **PROCESSING DEVICE AND METHOD, AIR POLLUTION DETECTION DEVICE AND METHOD**

VERARBEITUNGSVORRICHTUNG UND -VERFAHREN,
LUFTVERSCHMUTZUNGSNACHWEISVORRICHTUNG UND -VERFAHREN

DISPOSITIF ET PROCÉDÉ DE TRAITEMENT, DISPOSITIF DE DÉTECTION DE LA POLLUTION DE
L'AIR ET PROCÉDÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **09.10.2018 EP 18199364**

(43) Date of publication of application:
**15.04.2020 Bulletin 2020/16**

(73) Proprietor: **Sony Semiconductor Solutions**
**Corporation**
**Atsugi-shi, Kanagawa 243-0014 (JP)**

(72) Inventors:
• **Eslier, Thomas**
**70327 Stuttgart (DE)**
• **Siddiqui, Muhammad**
**70327 Stuttgart (DE)**

(74) Representative: **Witte, Weller & Partner**
**Patentanwälte mbB**
**Postfach 10 54 62**
**70047 Stuttgart (DE)**

(56) References cited:
**US-A1- 2006 266 950**

• **GUILLOT P: "Optical methods of remote sensing of atmospheric pollution", SPECTROCHIMICA ACTA. PART B: ATOMIC SPECTROSCOPY, NEW YORK, NY, US, US, vol. 38, no. 11-12, 1983, pages 1457-1464, XP026567318, ISSN: 0584-8547, DOI: 10.1016/0584-8547(83)80008-1 [retrieved on 1983-01-01]**
• **SEKINE S: "Spectral distributions of clear sky light and their chromaticities", J. LIGHT VISUAL ENVIRON. 15(1), 1991, pages 23-32, XP055663108, Retrieved from the Internet: URL:https://www.jstage.jst.go.jp/article/j lve/15/1/15_1_1_23/_pdf/-char/ja [retrieved on 2020-01-29]**
• **LÓPEZ-ÁLVAREZ M A ET AL: "Selecting algorithms, sensors, and linear bases for optimum spectral recovery of skylight", JOURNAL OF THE OPTICAL SOCIETY OF AMERICA A, vol. 24, no. 4, April 2007 (2007-04), page 942, XP055663082, US ISSN: 1084-7529, DOI: 10.1364/JOSAA.24.000942**
• **KATO Y: "A simple method for the detection of PM2.5 air pollutions using MODIS data", PROCEEDINGS OF SPIE VOL. 9876, REMOTE SENSING OF THE ATMOSPHERE, CLOUDS, AND PRECIPITATION VI, vol. 9876, 5 May 2016 (2016-05-05), pages 98762X-98762X, XP060069159, DOI: 10.1117/12.2223947 ISBN: 978-1-5106-1533-5**

EP 3 637 068 B1

**Description**

BACKGROUND

FIELD OF THE DISCLOSURE

The present disclosure relates to a processing device and a processing method for determining air pollution. The present disclosure relates further to an air pollution detection device and a corresponding air pollution detection method.

DESCRIPTION OF RELATED ART

Outdoor air quality analysis is of interest to understand atmospheric phenomena and external factors that affect air quality. Further, the need regarding the air pollution detection is increasing throughout the years because air quality has a high impact on people's life. A frequent exposure can increase illness, especially for children.

Conventionally, gas sensors using a chemical are used to detect the air pollution. The chemical used is depending on the target gas. For instance, the known MQ131 Ozone Gas Sensor (Low Concentration) uses tungsten trioxide. A chemical reaction is happening between the chemical itself and the target gas, which is e.g. ozone in case photochemical haze shall be detected.

Gas sensors have, however, various disadvantages, including the need to have a calibration over time, their reaction to all kind of oxides (nitrogen dioxide, carbon dioxide, etc.), their behavior to have a cold start (the amount of time is depending on the sensor) and to have a work cycle (heating - measurement - waiting), the sensitivity to humidity, and the difficulties to be integrated easily into a user device, such as a mobile phone, due to their size.

Hence, there is a need for another way of detecting air pollution, in particular photochemical haze, without the use of a gas sensor.

The "background" description provided herein is for the purpose of generally presenting the context of the disclosure. Work of the presently named inventor(s), to the extent it is described in this background section, as well as aspects of the description which may not otherwise qualify as prior art at the time of filing, are neither expressly or impliedly admitted as prior art against the present disclosure.

Prior art can be found in the following document:

GUILLOT P, "Optical methods of remote sensing of atmospheric pollution", SPECTROCHIMICA ACTA. PART B: ATOMIC SPECTROSCOPY, NEW YORK, NY, US, US, vol. 38, no. 11-12, doi:10.1016/0584-8547(83)80008-1, ISSN 0584-8547, (1983), pages 1457 - 1464.
US 2006/266950 A1.
SEKINE S, "Spectral distributions of clear sky light and their chromaticities", J. LIGHT VISUAL ENVIRON. 15(1), (1991), pages 23 - 32, URL: https://www.jstage.jst.go.jp/article/jlve/15/1/15 1 1 23/ pdf/-char/ja.
LÓPEZ-ÁLVAREZ M A ET AL, "Selecting algorithms, sensors, and linear bases for optimum spectral recovery of skylight", JOURNAL OF THE OPTICAL SOCIETY OF AMERICA A, US, (200704), vol. 24, no. 4, doi:10.1364/JOSAA.24.000942, ISSN 1084-7529, page 942.
KATO Y, "A simple method for the detection of PM2.5 air pollutions using MODIS data", PROCEEDINGS OF SPIE VOL. 9876, REMOTE SENSING OFTHE ATMOSPHERE, CLOUDS, AND PRECIPITATION VI, (20160505), vol. 9876, doi:10.1117/12.2223947, ISBN 978-1-5106-1533-5, pages 98762X- 98762X.

SUMMARY

It is an object to provide a processing device and a processing method as well as an air pollution detection device and a corresponding air pollution detection method, by which air pollution can be determined more easily and without the need of a gas sensor. It is a further object to provide as well as a corresponding computer program and a non-transitory computer-readable recording medium for implementing the processing method.

According to an aspect there is provided a processing device for determining air pollution as defined in claim 1.

According to a preferred embodiment, there is provided an air pollution detection device as defined in claim 8.

According to a further aspect, a processing method for determining air pollution is provided according to claim 7. According to a preferred embodiment, an air pollution detection method of claim 10 is provided. Furthermore, a computer program comprising program means for causing a computer to carry out the steps of the processing method disclosed herein, when said computer program is carried out on a computer, as well as a non-transitory computer-readable recording medium that stores therein a computer program product, which, when executed by a processor, causes the processing method disclosed herein to be performed are provided.

Embodiments are defined in the dependent claims. It shall be understood that the disclosed air pollution detection device, the disclosed methods, the disclosed computer program and the disclosed computer-readable recording medium have similar and/or identical further embodiments as the claimed processing device and as defined in the dependent claims and/or disclosed herein.

One of the aspects of the disclosure is to make use of a measurement of the solar spectrum which helps at detecting the outdoor air pollution and more specifically the presence of photochemical haze. By carrying measurements using a (stationary or mobile) spectrometer it is possible to observe a change in the solar spectrum as a function of the air pollution, particularly photochemical haze, which is a consequence of tropospheric ozone. The higher the tropospheric ozone concentration, the denser the photochemical haze is. The presence of photochemical haze has a similar effect on the solar spectrum as the presence of clouds (decrease Rayleigh scattering occurrence, increase Mie Scattering occurrence). Measurements of the solar spectrum are evaluated according to the present disclosure to detect the presence and, optionally, the level of air pollution.

The foregoing paragraphs have been provided by way of general introduction, and are not intended to limit the scope of the following claims. The described embodiments, together with further advantages, will be best understood by reference to the following detailed description taken in conjunction with the accompanying drawings.

BRIEF DESCRIPTION OF THE DRAWING

A more complete appreciation of the disclosure and many of the attendant advantages thereof will be readily obtained as the same becomes better understood by reference to the following detailed description when considered in connection with the accompanying drawings, wherein:

Fig. 1        shows a schematic diagram of a general air pollution detection device outside the scope of the appended claims but useful for understanding the invention,

Fig. 2        shows a flow chart of a general method outside the scope of the appended claims but useful for understanding the invention,

Fig. 3        shows a diagram illustrating some of the steps of themethod according to the present disclosure,

Fig. 4        shows a diagram of the normalized relative spectral power distribution over wavelength,

Fig. 5        shows a schematic diagram of an embodiment of an air pollution detection device according to the present disclosure,

Figs. 6A-6C   show diagrams of various signals used in the an air pollution detection device according to the present disclosure, and

Fig. 7        shows a diagram of the normalized value of different variables used for verification of the present disclosure.

DETAILED DESCRIPTION OF THE EMBODIMENTS

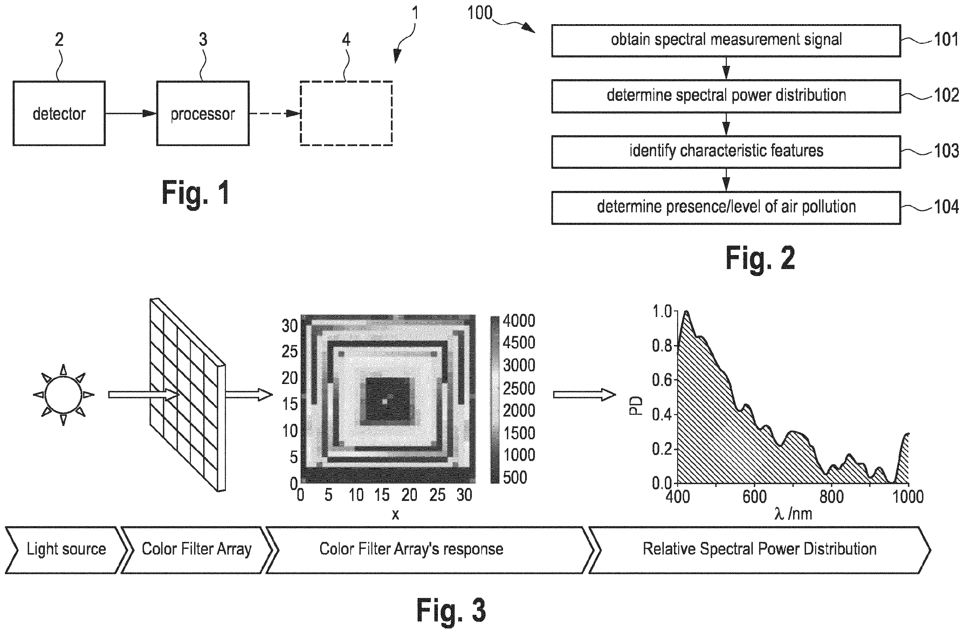

Referring now to the drawings, wherein like reference numerals designate identical or corresponding parts throughout the several views, Fig. 1 shows a schematic diagram of a general air pollution detection device 1 outside the scope of the appended claims but useful for understanding the invention.

The air pollution detection device 1 comprises a spectral measurement detector 2 and a processing device 3. The spectral measurement detector 2 is configured to detect sunlight in a spectral range of the solar spectrum and to generate a spectral measurement signal from the detected sunlight. In an exemplary embodiment a NanoLambda NSP32 Nano Spectrometer may be used as spectral measurement detector 2. The processing device 3 is configured to determine the presence and/or level of air pollution from the spectral measurement signal. The processing device 3 may e.g. be implemented by a programmed processor or computer. The result of the processing may e.g. be depicted on a display 4.

Fig. 2 shows a flow chart of a general method 100 outside the scope of the appended claims but useful for understanding the invention, and which is preferably carried out by the processing device 3. In a first step 101a spectral measurement signal representing sunlight measured in a spectral range of the solar spectrum is obtained (e.g. received or retrieved from the spectral measurement detector 2 or from a memory). In a second step 102 a spectral power distribution is determined from the obtained spectral measurement signal. In a third step 103 characteristic features of the spectral power distribution are identified in a wavelength range of violet, blue and/or green light. In a fourth step 104 the presence and/or level of air pollution is determined from the identified characteristic features.

The described solar spectrum-based detector and detection method help at detecting the outdoor air pollution and more specifically the presence of photochemical haze. Photochemical haze is a consequence of tropospheric ozone. It appears from the reaction of ozone with hydrocarbons. As soon as there are hydrocarbons (e.g. from car traffic) and ozone, there will be haze formation. Ozone is not visible to the human eye whereas the haze is a visible cloud (i.e. scattered light). Ground-level ozone is formed when hydrocarbons and nitrogen dioxide combine in the presence of

ultraviolet light:

NO$_2$ + ultraviolet light -> O + NO

O + O$_2$ -> O$_3$.

Fig. 3 shows a diagram illustrating some of the steps of a method according to the present disclosure. The use of the spectrometer 2 (such as a color filter array or a plasmonic filter) permits to acquire photons at wavelengths between 400 and 1000nm. For this purpose, the spectrometer 2 is facing the sky and is e.g. fixed on a flat surface in order to capture the direct and diffuse solar radiation. The measured signal (color filter response) is evaluated by generating and analyzing the relative spectral power distribution (PD).

A thin (pin-hole type) aperture may be used in front of the spectrometer 2 to limit the Field Of View (FOV) and to avoid saturation for obtaining solar spectrum information from the sky.

The photochemical haze has an impact on the solar spectrum. The effect on the solar spectrum is due to the fact that the haze is reducing Rayleigh scattering occurrence and increasing Mie scattering occurrence. The effect of the photochemical haze is similar as the effect of the clouds on the solar spectrum.

Reducing Rayleigh scattering occurrence can be formulated as

$$I = I_0 \frac{8\pi^4 \, \alpha^2}{\lambda^4 R^2} (1 + cos^2(\theta))$$

wherein $\alpha$ is the polarizability (e.g. vertically, horizontally, depolarized), $\lambda$ is the wavelength of the incoming photon, R is the distance from scattered and $\theta$ is the scattering angle. Rayleigh scattering is considered to be an elastic scattering meaning that no energy is lost in the scattering and the wavelength of the scattered light is the same as the incident light. It shall be noted that the intensity of the scattered photon is proportional to $1/\lambda^4$, which means that blue light will have a higher intensity than red light and the result is a blue sky at day time.

Increasing Mie scattering occurrence can be formulated as

$$J\{n, \theta\} = J_0 \frac{1}{\alpha^2} (i_1\{\alpha, n, \theta\} + i_2\{\alpha, n, \theta\})$$

wherein $i_1$ and $i_2$ are called intensity functions. $i_1$ corresponds to intensity of the polarized light in the horizontal light while $i_2$ is for the vertical plane. Also, n corresponds to the index of refraction, $\alpha$ is the size parameter, $J_0$ is the intensity of incident radiation. A major difference also with Rayleigh scattering, it is that Mie scattering is not strongly wavelength dependent. The scattering is proportional to $\frac{1}{\alpha^2}$ , where $\alpha$ is the radius of the scattered, times an intensity factor. Clouds are common causes of Mie Scattering. As well as the photochemical haze, that is why it is visible and the spectrometer can sense it.

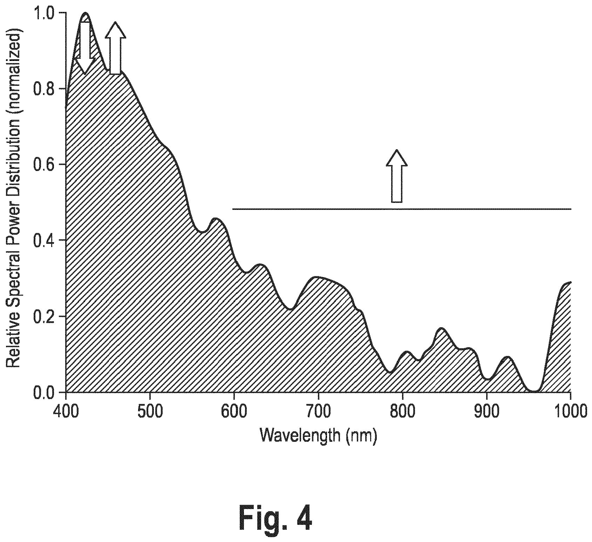

Fig. 4 shows a diagram of the normalized relative spectral power distribution over wavelength. Characteristic features of the normalized relative spectral power distribution that are evaluated according to the present disclosure include one or more of the frequency of a peak, the intensity of a peak, the number of peaks, and the area under the curve of peak (i.e. the integral value of the first Gaussian component. In an embodiment, changes of the spectral power distribution are compared to one or more earlier spectral power distributions and/or one or more reference spectral power distributions.

Hereby, the wavelength range of 400 nm to 500 nm is of particular interest. According to the invention, a Gaussian mixture of the spectral power distribution values at 425 nm and 475 nm is determined and used for determining the presence and/or level of air pollution, as defined in appended independent claims 1,7. A mean, variance and/or integral of the spectral power distribution values at 425 nm and 475 nm may be determined as statistical information, which may then be used for determining the presence and/or level of air pollution. For instance, if the spectral power distribution value at 425 nm falls below a first threshold it may be determined that air pollution is present. In addition or alternatively, it may be determined that the air pollution level is higher the lower the spectral power distribution value at 425 nm is. In another embodiment it may be determined that air pollution is present if the amplitude of the peak at 475 nm increases.

The level of air pollution may be determined by determining the amount (absolute or relative) by which the amplitude of the one or more peaks at 425 nm and/or 475 nm increases. Other options are to use the amount by which the mean, variance and/or integral of the spectral power distribution values at 425 nm and 475 nm increases.

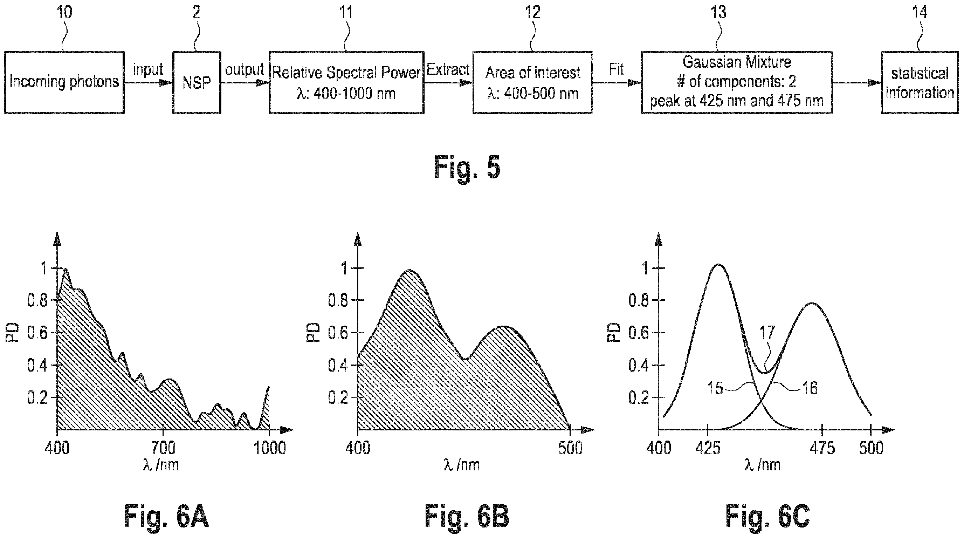

Fig. 5 shows a schematic diagram of an embodiment of an air pollution detection device according to the present disclosure and Figs. 6A-6C show diagrams of various signals used in an air pollution detection device according to the present disclosure. As explained above, the incoming photons 10 are measured by the spectral measurement detector 2. From the output of the spectral measurement detector 2 a relative spectral power distribution 11 (as shown in Fig. 6A) is determined (by the processing device 3), from which characteristic features 12 in the area of interest (400 to 500 nm; shown in Fig. 6B) are extracted. These are fitted to a Gaussian Mixture Model 13, using particularly the peaks at 425 nm and 475 nm. This also confirms the correlation between solar spectra and $O_3$. Statistical information 14, such as the mean, integral and/or variance of the two components at 425 nm and 475 nm can thus be determined and used for determining if air pollution is present and, optionally, the level of air pollution. Fig. 6C shows the two components 15, 16 and the Gaussian fit 17.

The characteristic features 12 in the area of interest may hereby be fitted to a Gaussian Mixture Model by fitting the relative spectral power distribution in this wavelength domain by two Gaussian components. The fitting may be done using an Expectation-Maximization algorithm for fitting Gaussian components. The two peaks have Gaussian shapes, which is one reason to use these peaks fit using Gaussian components. Further, in this way relevant statistical information about the peaks can be obtained and expected.

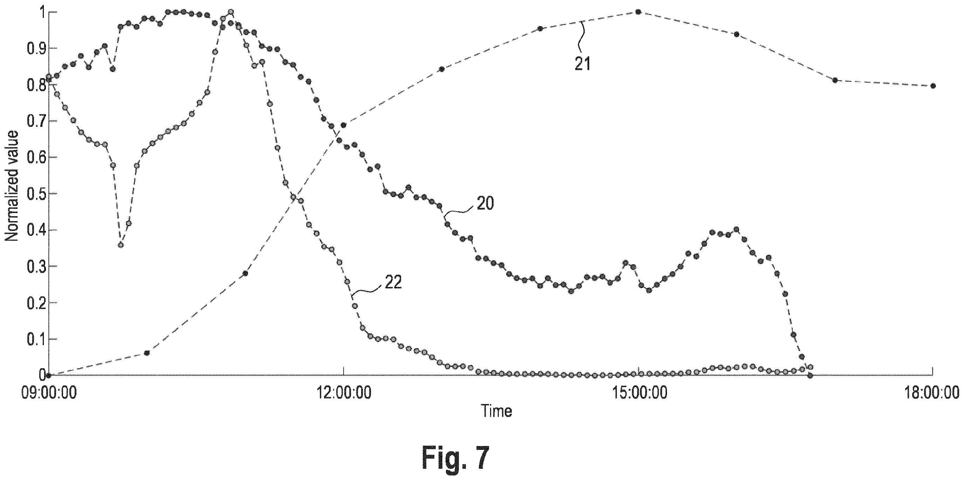

Fig. 7 shows a diagram of the normalized value of different variables used for verification of the present disclosure for an exemplary measurement. Curve 20 represents the means of the first Gaussian mixture model component, i.e. the component of the relative spectral power distribution at 425 nm. Curve 21 represents the ozone concentration as measured by a weather station. Curve 22 represents a gas sensor output. A low value of the first curve 20 means a high air pollution and a high value of the curve 20 means a low air pollution (i.e. clean air). Hence, the integral value of the Gaussian component of the first peak and the ozone concentration have opposite phase. The integral value of the Gaussian component of the second peak and the ozone concentration are in-phase with each other.

The air pollution detection device 1 may be implemented as a separate device, e.g. a small, cheap, mobile user device, or as a stationary device, or may be integrated into another device, e.g. into a pollution measurement station. In an embodiment, the spectral measurement detector 2 may e.g. be provided as additional sensor (or as alternative to an image sensor) in a user device, such as a mobile phone.

The measurement information may be processed and used by the user of the device, or may be transmitted to a central station, e.g. the environmental authority of a city, or to a database in the cloud, to which various user or applications may have access. The processing of the measurement information may thus be done in the user device or centrally in an central station. The providers of street maps, such as Google maps, may e.g. then use the resulting data to provide indications of air pollution in street maps or in separate air pollution maps.

Mobile spectrometers may further be used as or in IoT (Internet of Things) compatible device. It can be considered as a 'Thing' in a sense that, it is mobile, it is possible to exchange the data coming from it through the internet without human intervention, and it has a low-power consumption.

An artificial neural network may be used to create an algorithm able to recognize when the spectrometer is working under a clean environment from a highly polluted one. A Wireless Sensor Network (WSN) system is one of the applications. Building such a network can help covering a large surface of the atmosphere. The larger the network, the better the coverage is and so the quality of the output. If built, a neural network can train itself by using new incoming user inputs.

The advantages are that embedded spectrometers do not need a calibration, do not react to oxides and have a limited environment-dependency (limited by the use of an aperture), do not have cold start (the amount of time is depending on the sensor), do not have a work cycle (heating - measurement - waiting), and can be integrated within existing IoT device for masses (i.e. mobile phones).

Thus, the foregoing discussion discloses and describes merely exemplary embodiments of the present disclosure. As will be understood by those skilled in the art, the present disclosure may be embodied in other specific forms, limited only by the scope of the appended claims. Accordingly, the disclosure of the present disclosure is intended to be illustrative. The invention is defined and limited by the appended claims. The disclosure, including any readily discernible variants of the teachings herein, defines, in part, the scope of the foregoing claim terminology such that no inventive subject matter is dedicated to the public.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

In so far as embodiments of the disclosure have been described as being implemented, at least in part, by software-controlled data processing apparatus, it will be appreciated that a non-transitory machine-readable medium carrying such software, such as an optical disk, a magnetic disk, semiconductor memory or the like, is also considered to represent an embodiment of the present disclosure. Further, such a software may also be distributed in other forms, such as via the Internet or other wired or wireless telecommunication systems.

The elements of the disclosed devices, apparatus and systems may be implemented by corresponding hardware and/or software elements, for instance appropriated circuits. A circuit is a structural assemblage of electronic components including conventional circuit elements, integrated circuits including application specific integrated circuits, standard integrated circuits, application specific standard products, and field programmable gate arrays. Further a circuit includes central processing units, graphics processing units, and microprocessors which are programmed or configured according to software code. A circuit does not include pure software, although a circuit includes the above-described hardware executing software.

**Claims**

1.   Processing device for determining air pollution, said processing device comprising processing circuitry configured to

         - obtain a spectral measurement signal representing sunlight measured in a spectral range of the solar spectrum,
         - determine a spectral power distribution from the obtained spectral measurement signal,
         - identify characteristic features of the spectral power distribution in a wavelength range of violet, blue and/or green light,

     **characterized in that** the processing circuitry is configured to

         - determine the presence and/or level of air pollution from the identified characteristic features by determining a Gaussian mixture of the spectral power distribution values at 425 nm and 475 nm and using the Gaussian mixture for determining the presence and/or level of air pollution,

     and **in that** the processing circuitry is further configured

         - to determine that air pollution is present if the Gaussian mixture of the spectral power distribution value at 425 nm falls below a first threshold, and/or
         - to determine that the air pollution level is higher the lower the Gaussian mixture of the spectral power distribution value at 425 nm is, and/or
         - to determine that air pollution is present if an amplitude of a peak of the Gaussian mixture of the spectral power distribution at 475 nm increases, and/or
         - to determine the level of air pollution by determining the absolute and/or relative amount by which an amplitude of one or more peaks of the Gaussian mixture of the spectral power distribution at 425 nm and/or 475 nm changes, and/or the amount by which the mean, variance and/or integral of the Gaussian mixture of the spectral power distribution values at 425 nm and 475 nm changes.

2.   Processing device as claimed in claim 1,
     wherein the processing circuitry is further configured to identify as characteristic features one or more of the frequency of a peak, the intensity of a peak, the number of peaks, and the area under the curve of peak.

3.   Processing device as claimed in claim 1,
     wherein the processing circuitry is further configured to identify characteristic features of the Gaussian mixture of the spectral power distribution by identifying changes of the Gaussian mixture of the spectral power distribution compared to one or more earlier spectral power distributions and/or one or more reference spectral power distributions.

4.   Processing device as claimed in claim 1,
     wherein the processing circuitry is further configured to identify characteristic features of the Gaussian mixture of the spectral power distribution in a wavelength range of 400 nm to 500 nm.

5.   Processing device as claimed in claim 4,
     wherein the processing circuitry is further configured to determine as statistical information a mean, variance and/or integral of the Gaussian mixture of the spectral power distribution values at 425 nm and 475 nm and to use the statistical information for determining the presence and/or level of air pollution.

6.   Processing device as claimed in claim 1,

wherein the processing circuitry is configured to determine the presence and/or level of photochemical haze from the identified characteristic features.

7. Processing method for determining air pollution, said processing method comprising:

   - obtaining a spectral measurement signal representing sunlight measured in a spectral range of the solar spectrum,
   - determining a spectral power distribution from the obtained spectral measurement signal,
   - identifying characteristic features of the spectral power distribution in a wavelength range of violet, blue and/or green light,

   **characterized in that** the method comprises

   - determining the presence and/or level of air pollution from the identified characteristic features by determining a Gaussian mixture of the spectral power distribution values at 425 nm and 475 nm and using the Gaussian mixture for determining the presence and/or level of air pollution,

   and **in that** the processing method further comprises

   - determining that air pollution is present if the Gaussian mixture of the spectral power distribution value at 425 nm falls below a first threshold, and/or
   - determining that the air pollution level is higher the lower the Gaussian mixture of the spectral power distribution value at 425 nm is, and/or
   - determining that air pollution is present if an amplitude of a peak of the Gaussian mixture of the spectral power distribution at 475 nm increases, and/or
   - determining the level of air pollution by determining the absolute and/or relative amount by which an amplitude of one or more peaks of the Gaussian mixture of the spectral power distribution at 425 nm and/or 475 nm changes, and/or the amount by which the mean, variance and/or integral of the Gaussian mixture of the spectral power distribution values at 425 nm and 475 nm changes.

8. Air pollution detection device comprising:

   - a spectral measurement detector configured to detect sunlight in a spectral range of the solar spectrum and to generate a spectral measurement signal from the detected sunlight, and
   - a processing device as claimed in claim 1 for determining the presence and/or level of air pollution from the spectral measurement signal.

9. Air pollution detection device as claimed in claim 8,
   wherein the spectral measurement detector comprises a color filter array or a plasmonic filter.

10. Air pollution detection method comprising:

   - detecting sunlight in a spectral range of the solar spectrum and to generate a spectral measurement signal from the detected sunlight, and
   - a processing method as claimed in claim 7 for determining the presence and/or level of air pollution from the spectral measurement signal.

11. A computer program comprising program code means for causing a computer to perform the steps of said method according to claim 7 when said computer program is carried out on a computer.


**Patentansprüche**

1. Verarbeitungsvorrichtung zum Bestimmen der Luftverschmutzung, wobei die Verarbeitungsvorrichtung eine Verarbeitungsschaltung umfasst, die für Folgendes ausgebildet ist:

   - Erhalten eines Spektralmesssignals, das das in einem Spektralbereich des Sonnenspektrums gemessene Sonnenlicht repräsentiert,

- Bestimmen einer Spektralleistungsverteilung aus dem erhaltenen Spektralmesssignal,
- Identifizieren charakteristischer Merkmale der Spektralleistungsverteilung in einem Wellenlängenbereich von violettem, blauem und/oder grünem Licht,

**dadurch gekennzeichnet, dass** die Verarbeitungsschaltung für Folgendes ausgebildet ist:

- Bestimmen des Vorhandenseins und/oder des Grades der Luftverschmutzung aus den identifizierten charakteristischen Merkmalen, indem eine Gaußsche Mischung der Spektralleistungsverteilungswerte bei 425 nm und 475 nm bestimmt wird und die Gaußsche Mischung zur Bestimmung des Vorhandenseins und/oder des Grades der Luftverschmutzung verwendet wird,

und dadurch, dass die Verarbeitungsschaltung ferner für Folgendes ausgebildet ist:

- Bestimmen, dass eine Luftverschmutzung vorliegt, wenn die Gaußsche Mischung des Spektralleistungsverteilungswertes bei 425 nm unter einen ersten Schwellenwert fällt, und/oder
- Bestimmen, dass der Luftverschmutzungsgrad umso höher ist, je niedriger die Gaußsche Mischung des Spektralleistungsverteilungswertes bei 425 nm ist, und/oder
- Bestimmen, dass eine Luftverschmutzung vorliegt, wenn eine Amplitude eines Peaks der Gaußschen Mischung der Spektralleistungsverteilung bei 475 nm zunimmt, und/oder
- Bestimmen des Grads der Luftverschmutzung, indem der absolute und/oder relative Betrag bestimmt wird, um den sich eine Amplitude eines oder mehrerer Peaks der Gaußschen Mischung der Spektralleistungsverteilung bei 425 nm und/oder 475 nm ändert, und/oder der Betrag, um den sich der Mittelwert, die Varianz und/oder das Integral der Gaußschen Mischung der Spektralleistungsverteilungswerte bei 425 nm und 475 nm ändert.

2. Verarbeitungsvorrichtung nach Anspruch 1,
wobei die Verarbeitungsschaltung ferner so ausgebildet ist, dass sie als charakteristische Merkmale eines oder mehrere der folgenden Merkmale identifiziert: die Frequenz eines Peaks, die Intensität eines Peaks, die Anzahl der Peaks und die Fläche unter der Kurve des Peaks.

3. Verarbeitungsvorrichtung nach Anspruch 1,
wobei die Verarbeitungsschaltung ferner so ausgebildet ist, dass sie charakteristische Merkmale der Gaußschen Mischung der Spektralleistungsverteilung identifiziert, indem sie Änderungen der Gaußschen Mischung der Spektralleistungsverteilung im Vergleich zu einer oder mehreren früheren Spektralleistungsverteilungen und/oder einer oder mehreren Referenz-Spektralleistungsverteilungen identifiziert.

4. Verarbeitungsvorrichtung nach Anspruch 1,
wobei die Verarbeitungsschaltung ferner so ausgebildet ist, dass sie charakteristische Merkmale der Gaußschen Mischung der Spektralleistungsverteilung in einem Wellenlängenbereich von 400 nm bis 500 nm identifiziert.

5. Verarbeitungsvorrichtung nach Anspruch 4,
wobei die Verarbeitungsschaltung ferner so ausgebildet ist, dass sie als statistische Information einen Mittelwert, eine Varianz und/oder ein Integral der Gaußschen Mischung der Spektralleistungsverteilungswerte bei 425 nm und 475 nm bestimmt und die statistische Information zur Bestimmung des Vorhandenseins und/oder des Grades der Luftverschmutzung verwendet.

6. Verarbeitungsvorrichtung nach Anspruch 1,
wobei die Verarbeitungsschaltung so ausgebildet ist, dass sie aus den identifizierten charakteristischen Merkmalen das Vorhandensein und/oder den Grad der photochemischen Trübung bestimmt.

7. Verfahren zur Bestimmung der Luftverschmutzung, wobei das Verfahren Folgendes umfasst:

- Erhalten eines Spektralmesssignals, das das in einem Spektralbereich des Sonnenspektrums gemessene Sonnenlicht repräsentiert,
- Bestimmen einer Spektralleistungsverteilung aus dem erhaltenen Spektralmesssignal,
- Identifizieren charakteristischer Merkmale der Spektralleistungsverteilung in einem Wellenlängenbereich von violettem, blauem und/oder grünem Licht,

**dadurch gekennzeichnet, dass** das Verfahren Folgendes umfasst:

- Bestimmen des Vorhandenseins und/oder des Grades der Luftverschmutzung aus den identifizierten charakteristischen Merkmalen durch Bestimmen einer Gaußschen Mischung der Spektralleistungsverteilungswerte bei 425 nm und 475 nm und Verwenden der Gaußschen Mischung zum Bestimmen des Vorhandenseins und/oder des Grades der Luftverschmutzung,

und dadurch, dass das Verarbeitungsverfahren ferner Folgendes umfasst:

- Bestimmen, dass eine Luftverschmutzung vorliegt, wenn die Gaußsche Mischung des Spektralleistungsverteilungswertes bei 425 nm unter einen ersten Schwellenwert fällt, und/oder
- Bestimmen, dass der Luftverschmutzungsgrad umso höher ist, je niedriger der Wert der Gaußschen Mischung der Spektralleistungsverteilung bei 425 nm ist, und/oder
- Bestimmen, dass eine Luftverschmutzung vorliegt, wenn eine Amplitude eines Peaks der Gaußschen Mischung der Spektralleistungsverteilung bei 475 nm zunimmt, und/oder
- Bestimmen des Luftverschmutzungsgrades durch Bestimmen des absoluten und/oder relativen Betrags, um den sich eine Amplitude eines oder mehrerer Peaks des Gaußschen Gemischs der Spektralleistungsverteilung bei 425 nm und/oder 475 nm ändert, und/oder des Betrags, um den sich der Mittelwert, die Varianz und/oder das Integral des Gaußschen Gemischs der Spektralleistungsverteilung ändert.

8.  Luftverschmutzungs-Erkennungsvorrichtung, die Folgendes umfasst:

- einen Spektralmessungsdetektor, der so ausgebildet ist, dass er Sonnenlicht in einem Spektralbereich des Sonnenspektrums erkennt und ein Spektralmesssignal aus dem erkannten Sonnenlicht erzeugt, und
- eine Verarbeitungsvorrichtung nach Anspruch 1 zum Bestimmen des Vorhandenseins und/oder des Grades der Luftverschmutzung aus dem Spektralmesssignal.

9.  Luftverschmutzungs-Erkennungsvorrichtung nach Anspruch 8,
    wobei der Spektralmessungsdetektor eine Farbfilteranordnung oder einen plasmonischen Filter umfasst.

10. Luftverschmutzungs-Erkennungsverfahren, das Folgendes umfasst:

- Erkennen von Sonnenlicht in einem Spektralbereich des Sonnenspektrums und Erzeugen eines Spektralmesssignals aus dem erkannten Sonnenlicht, und
- ein Verarbeitungsverfahren nach Anspruch 7 zum Bestimmen des Vorhandenseins und/oder des Grades der Luftverschmutzung aus dem Spektralmesssignal.

11. Computerprogramm, das Programmcodemittel enthält, die einen Computer veranlassen, die Schritte des Verfahrens nach Anspruch 7 durchzuführen, wenn das Computerprogramm auf einem Computer ausgeführt wird.

**Revendications**

1.  Dispositif de traitement pour déterminer une pollution atmosphérique, ledit dispositif de traitement comprenant des circuits de traitement configurés pour

- obtenir un signal de mesure spectrale représentant la lumière solaire mesurée dans une plage spectrale du spectre solaire,
- déterminer une distribution de puissance spectrale à partir du signal de mesure spectrale obtenu,
- identifier des aspects caractéristiques de la distribution de puissance spectrale dans une gamme de longueurs d'onde de lumière violette, bleue et/ou verte,

**caractérisé en ce que** les circuits de traitement sont configurés pour

- déterminer la présence et/ou le niveau d'une pollution atmosphérique à partir des aspects caractéristiques identifiés en déterminant un mélange gaussien des valeurs de distribution de puissance spectrale à 425 nm et 475 nm et en utilisant le mélange gaussien pour déterminer la présence et/ou le niveau de pollution atmosphérique,

et **en ce que** les circuits de traitement sont configurés en outre pour

- déterminer la présence d'une pollution atmosphérique si le mélange gaussien de la valeur de distribution de puissance spectrale à 425 nm chute en dessous d'un premier seuil, et/ou

- déterminer que plus le mélange gaussien de la valeur de distribution de puissance spectrale à 425 nm est faible, plus le niveau de pollution atmosphérique est élevé, et/ou

- déterminer la présence d'une pollution atmosphérique si une amplitude d'un pic du mélange gaussien de distribution de puissance spectrale à 475 nm augmente, et/ou

- déterminer le niveau de pollution atmosphérique en déterminant la quantité absolue et/ou relative de variation d'une amplitude d'un ou de plusieurs pics du mélange gaussien de distribution de puissance spectrale à 425 nm et/ou 475 nm et/ou la quantité de variation de la moyenne, de la variance et/ou de l'intégrale du mélange gaussien des valeurs de distribution de puissance spectrale à 425 nm et 475 nm.

2. Dispositif de traitement selon la revendication 1,
dans lequel les circuits de traitement sont configurés en outre pour identifier comme aspects caractéristiques au moins la fréquence d'un pic, l'intensité d'un pic, le nombre de pics et l'aire sous la courbe de crête.

3. Dispositif de traitement selon la revendication 1,
dans lequel les circuits de traitement sont configurés en outre pour identifier des aspects caractéristiques du mélange gaussien de distribution de puissance spectrale en identifiant des variations du mélange gaussien de distribution de puissance spectrale par rapport à une ou plusieurs distributions de puissance spectrale antérieures et/ou une ou plusieurs distributions de puissance spectrale de référence.

4. Dispositif de traitement selon la revendication 1,
dans lequel les circuits de traitement sont configurés en outre pour identifier des aspects caractéristiques du mélange gaussien de distribution de puissance spectrale dans une gamme de longueurs d'onde de 400 nm à 500 nm.

5. Dispositif de traitement selon la revendication 4,
dans lequel les circuits de traitement sont configurés en outre pour déterminer comme informations statistiques une moyenne, une variance et/ou une intégrale du mélange gaussien de valeurs de distribution de puissance spectrale à 425 nm et 475 nm et utiliser les informations statistiques afin de déterminer la présence et/ou le niveau d'une pollution atmosphérique.

6. Dispositif de traitement selon la revendication 1,
dans lequel les circuits de traitement sont configurés en outre pour déterminer la présence et/ou le niveau d'une brume photochimique à partir des aspects caractéristiques identifiés.

7. Procédé de traitement pour déterminer une pollution atmosphérique, ledit procédé de traitement comprenant :

- l'obtention d'un signal de mesure spectrale représentant la lumière solaire mesurée dans une gamme spectrale du spectre solaire,

- la détermination d'une distribution de puissance spectrale à partir du signal de mesure spectrale obtenu,

- l'identification d'aspects caractéristiques de la distribution de puissance spectrale dans une gamme de longueurs d'onde de lumière violette, bleue et/ou verte,

**caractérisé en ce que** le procédé comprend la détermination de la présence et/ou du niveau de pollution atmosphérique à partir des aspects caractéristiques identifiés en déterminant un mélange gaussien de valeurs de distribution de puissance spectrale à 425 nm et 475 nm et en utilisant le mélange gaussien pour déterminer la présence et/ou le niveau de pollution atmosphérique,

et **en ce que** le procédé de traitement comprend en outre

- la détermination de la présence d'une pollution atmosphérique si le mélange gaussien de la valeur de distribution de puissance spectrale à 425 nm chute en dessous d'un premier seuil et/ou

- la détermination que plus le mélange gaussien de la valeur de distribution de puissance spectrale à 425 nm est faible, plus le niveau de pollution atmosphérique est élevé, et/ou

- la détermination de la présence d'une pollution atmosphérique si une amplitude d'un pic du mélange gaussien de distribution de puissance spectrale à 475 nm augmente, et/ou

- la détermination du niveau de pollution atmosphérique en déterminant la quantité absolue et/ou relative de variation d'une amplitude d'un ou de plusieurs pics du mélange gaussien de distribution de puissance spectrale à 425 nm et/ou 475 nm et/ou la quantité de variation de la moyenne, de la variance et/ou de l'intégrale du mélange gaussien des valeurs de distribution de puissance spectrale à 425 nm et 475 nm.

**8.** Dispositif de détection de pollution atmosphérique comprenant :

- un détecteur de mesure spectrale configuré pour détecter la lumière solaire dans une gamme spectrale du spectre solaire et générer un signal de mesure spectrale à partir de la lumière solaire détectée, et
- un dispositif de traitement selon la revendication 1 pour déterminer la présence et/ou le niveau d'une pollution atmosphérique à partir du signal de mesure spectrale.

**9.** Dispositif de détection de la pollution atmosphérique selon la revendication 8,
dans lequel le détecteur de mesure spectrale comprend un réseau de filtres couleur ou un filtre plasmonique.

**10.** Procédé de détection de pollution atmosphérique comprenant :

- la détection d'une lumière solaire dans une gamme spectrale du spectre solaire et la génération d'un signal de mesure spectrale à partir de la lumière solaire détectée, et
- un procédé de traitement selon la revendication 7 pour déterminer la présence et/ou le niveau de pollution atmosphérique à partir du signal de mesure spectrale.

**11.** Programme d'ordinateur comprenant un code de programme pour amener un ordinateur à réaliser les étapes dudit procédé selon la revendication 7 lorsque ledit programme d'ordinateur est exécuté sur un ordinateur.

Fig. 1

Fig. 2

- 101 obtain spectral measurement signal
- 102 determine spectral power distribution
- 103 identify characteristic features
- 104 determine presence/level of air pollution

Fig. 3

Light source | Color Filter Array | Color Filter Array's response | Relative Spectral Power Distribution

Fig. 4

**Fig. 5**

**Fig. 6A**

**Fig. 6B**

**Fig. 6C**

EP 3 637 068 B1

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006266950 A1

**Non-patent literature cited in the description**

- **GUILLOT P.** Optical methods of remote sensing of atmospheric pollution. *SPECTROCHIMICA ACTA. PART B: ATOMIC SPECTROSCOPY,* 1983, vol. 38 (11-12), ISSN 0584-8547, 1457-1464
- **SEKINE S.** Spectral distributions of clear sky light and their chromaticities. *J. LIGHT VISUAL ENVIRON,* 1991, vol. 15 (1), 23-32, https://www.jstage.jst.go.jp/article/jlve/15/1/15 1 1 23/pdf/-char/ja
- **LÓPEZ-ÁLVAREZ M A et al.** Selecting algorithms, sensors, and linear bases for optimum spectral recovery of skylight. *JOURNAL OF THE OPTICAL SOCIETY OF AMERICA A,* April 2007, vol. 24 (4), ISSN 1084-7529, 942
- **KATO Y.** A simple method for the detection of PM2.5 air pollutions using MODIS data. *PROCEEDINGS OF SPIE VOL. 9876, REMOTE SENSING OF THE ATMOSPHERE, CLOUDS, AND PRECIPITATION VI,* 05 May 2016, vol. 9876, ISBN 978-1-5106-1533-5, 98762X-98762X